(19) Europäisches Patentamt
European Patent Office
Office européen des brevets

(11) EP 0 719 539 A2

(12) **EUROPEAN PATENT APPLICATION**

(43) Date of publication:
**03.07.1996 Bulletin 1996/27**

(51) Int. Cl.[6]: **A61K 7/48**, C08G 77/46, B01F 17/00

(21) Application number: **95309384.6**

(22) Date of filing: **21.12.1995**

(84) Designated Contracting States:
**DE FR GB IT**

(30) Priority: **29.12.1994 GB 9426313**

(71) Applicant: **DOW CORNING S.A.**
**B-7180 Seneffe (BE)**

(72) Inventors:
- **De Smedt, Anne**
**B-6210 Reves (BE)**
- **Stoller, Catherine**
**B-1420 Briane l'Alleud (BE)**
- **Van Reeth, Isabelle Michelle Elisabeth**
**B-1435 Pietrebais (BE)**
- **Vincent, Anne-Marie**
**B-6210 Frasnes Lez Gosselies (BE)**

(74) Representative: **Vandamme, Luc Johan Roger**
**Dow Corning Limited,**
**Cardiff Road**
**Barry, South Glamorgan CF63 2YL, Wales (GB)**

(54) **Water in oil emulsifiers and emulsions made with them**

(57) An organopolysiloxane polyoxyalkylene copolymer of the formula

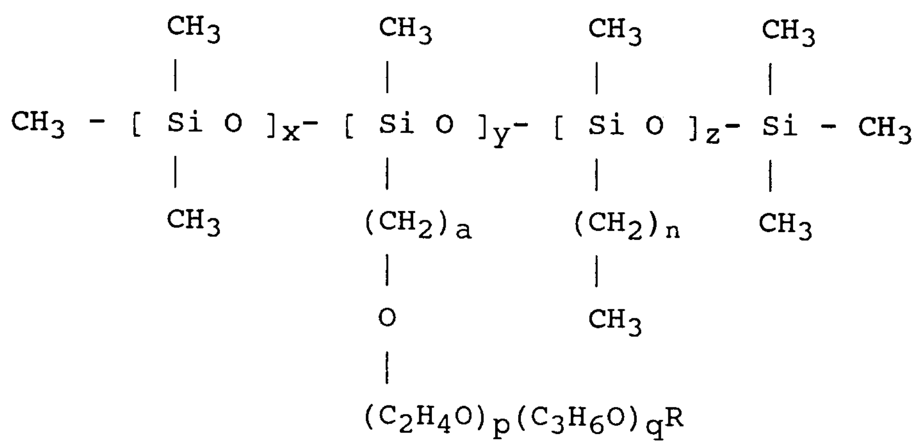

(I)

wherein R is H or $C_{1-4}$ alkyl, x, y and z are at least 1, x+y+z is from 20 to 150, 3y is greater than x, a is 1 to 3, p is at least 1 and q is 0 or an integer, p and q are such that $\overline{MW}$ of $(C_2H_4O)_p(C_3H_6O)_qR$ is no more than 500 and n is 7 to 23. Also disclosed are water-in-oil emsulsions, especially with polar oils.

EP 0 719 539 A2

## Description

Water-in-oil emulsions have long been the subject of interest for the cosmetic industry. They offer a number of advantages over oil-in-water emulsions both to the consumer of cosmetic products and to the manufacturer. For example, where applied to skin-care products water-in-oil emulsions enhance the skin's natural moisturisation process, form water-resistant films, provide better resistance to wash-off and an increased efficacy of the oil-soluble active ingredients. As water-in-oil emulsions are stable with an amount of water which exceeds 50% of the total emulsion, they also provide the opportunity to reduce the cost of the production, even if the emulsifiers themselves may be more expensive than those which are suitable for oil-in-water emulsions.

There are however also a number of constraints to water-in-oil emulsion systems. They tend to be more difficult to manufacture, have decreased stability where the internal phase is greater than 60% by weight of the total emulsion, affecting the internal phase volume by distortion of the aqueous phase droplets.

Many of the cosmetic water-in-oil products have been based on mineral oils and petrolatum as the oil phase, because these tend to be of lower cost and more easy to formulate. However, the use of mineral oil as external phase of cosmetic products is no longer favoured to the same extent in the cosmetic industry. Because of market trends, there is a desire to use more polar organic oils, e.g. vegetable oils and triglycerides instead of the mineral oil based materials.

However, conventional organic emulsifiers are not suitable for incorporating such oils at a higher level than about 10% by weight of the total formula. Traditional silicone based emulsifiers also do not seem to give satisfactory results with these polar components. There is therefore a need to provide emulsifiers which will allow the use of higher levels of polar organic components, while maintaining a stable water-in-oil emulsion, especially for use with polar oils.

We have now found that certain novel silicone based emulsifiers are able to provide a solution.

Many silicone-based emulsifiers for water-in-oil emulsions are known. In US specification 4,532,132, there are disclosed organopolysiloxanes having the formula

$$Z-\underset{CH_3}{\overset{CH_3}{\underset{|}{\overset{|}{Si}}}}O-[\underset{CH_3}{\overset{CH_3}{\underset{|}{\overset{|}{Si}}}}O]_x-[\underset{R}{\overset{CH_3}{\underset{|}{\overset{|}{Si}}}}O]_y-[\underset{R'Q}{\overset{CH_3}{\underset{|}{\overset{|}{Si}}}}O]_z-\underset{CH_3}{\overset{CH_3}{\underset{|}{\overset{|}{Si}}}}-Z$$

wherein R denotes an alkyl group having from 6 to 16 carbon atoms, R' is an alkylene linking group, Q is a polyoxyalkylene radical of the formula $-(OCH_2CH_2)_p(OCHCH_3CH_3)_qOR''$ wherein R" is a hydrogen or lower alkyl group, Z denotes a monovalent radical, selected from $C_{1-5}$ hydrocarbons, R'Q groups and R groups, there being at least one R'Q group and one R group per molecule, the average values of $\underline{x}$, $\underline{y}$, $\underline{z}$, $\underline{p}$ and $\underline{q}$ being such that $\underline{p}\geqq\underline{q}$, $\underline{p}+\underline{q}$ has a value sufficient to provide a radical weight for Q of from 600 to 3500, $\underline{x}\leqq 3\underline{y}$, $\underline{x}+\underline{y}+\underline{z}$ has a value of from 30 to 400 and the total weight of Q radicals in the organopolysiloxane does not exceed a value of about 1/3 of the total weight of the organopolysiloxane. These emulsifiers are specifically stated to be useful for the preparation of water-in-mineral oil emulsions.

In US specification 4,698,178 there is provided a emulsifier for the preparation of water-in-silicone oil emulsions, which has the average formula

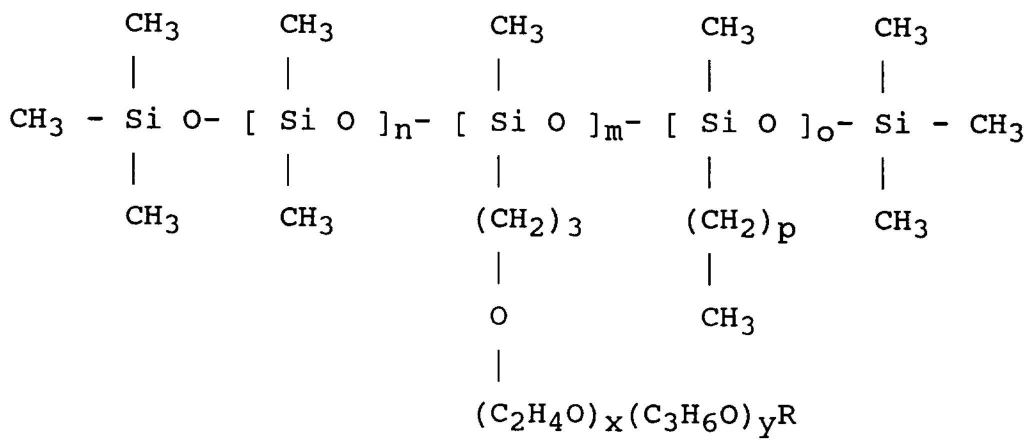

wherein R is an C1-4 alkyl radical or hydrogen, n is 10 to 200, m is 1 to 25, o is 1 to 100, provided o is not less than m and 3o is less than n, p is 7 to 17, and wherein the molecular weight of the $(C_2H_4O)_x(C_3H_6O)_yR$ group is 250 to 2000 with x and y selected so that the weight ratio of oxyethylene to oxypropylene is 100:0 to 20:80.

In EP specification 537,003, there is provided a silicone polyether alkyl copolymer water-in-oil emulsifier of the general formula $MD'_xD''_yM$, wherein M represents trimethylsiloxane units, D' represents alkyl siloxy units of the formula $(CH_3)R'SiO_{2/2}$ where R' is an alkyl group having from 6 to 30 carbon atoms, D" is an oxyalkylene siloxy group of the formula $(CH_3)R^2SiO_{2/2}$ where $R^2$ is a polyoxyalkylene ether residue of the formula $-(R^4)_p-(OR^3)_n-OR^5$ wherein each individual $R^3$ is an alkylene radical having from 2 to 6 carbon atoms, $R^4$ is an alkylene radical having from 2 to 20 carbon atoms, $R^5$ is hydrogen or a hydrocarbon radical of from 1 to 12 carbon atoms, n has a value of from about 5 to about 20 and p has a value of 0 or 1, x is from 1 to 29, y is from 1 to 29 with the proviso that x+y is from 2 to 30. In the preferred emulsions, a mineral oil is always present.

EP specification 540,199 discloses stable water-in-oil emulsions using as emulsifier a polyorganosiloxane polyoxyalkylene copolymer surface active agent of the formula $MD_xD'_yM$, wherein M represents trimethylsiloxane units, D represents siloxy units of the formula $R_2SiO_{2/2}$ where R is hydrogen or a substituted or unsubstituted hydrocarbon radical of from 1 to 12 carbon atoms, D' is an oxyalkylene siloxy group of the formula $RR'SiO_{2/2}$ where R' is a polyoxyalkylene ether residue of the formula $-(R^3)_a-(OR^2)_n-OR^4$ wherein $R^2$ is a $-CH_2CH_2-$ group, $R^3$ is an alkylene radical having from 1 to 20 carbon atoms, $R^4$ is the same as R, n has a value of from about 5 to about 20 and a has a value of 0 or 1, x is from 335 to 475, y is from 4 to 23 with the proviso that the molecular weight of units D is from 25,000 to 35,000 and the weight ratio of D to D' is from greater than 6/4 to 9/1. These surfactants are only used in conjunction with organic surfactants having a HLB value of from 8 to 18.

According to the present invention, there is provided a organopolysiloxane polyoxyalkylene copolymer of the general formula

```
                CH3           CH3           CH3         CH3
                 |             |             |           |
   CH3 - [ Si O ]x- [ Si O ]y- [ Si O ]z- Si - CH3     (I)
                 |             |             |           |
                CH3          (CH2)a        (CH2)n       CH3
                               |             |
                               O            CH3
                               |
                          (C2H4O)p(C3H6O)qR
```

wherein R denotes a hydrogen atom or a $C_{1-4}$ alkyl group, $x$, $y$ and $z$ have a value of at least 1, provided that $x+y+z$ has a value of from 20 to 150, 3$y$ must be greater than $x$, $a$ has a value of from 1 to 3, $p$ has a value of at least 1 and $q$ is 0 or an integer, provided that $p$ and $q$ are such that the mole-cular weight of $(C_2H_4O)_p(C_3H_6O)_qR$ is no more than 500 and $n$ has a value of from 7 to 23.

It is important that all the conditions of the structure as identified are fulfilled in order to ensure the improved ability to make water-in-oil emulsions with polar oils. It is particularly preferred that $a$ has a value of 3, $q$ has a value of 0, $p$ has a value of from 4 to 15, more preferably 5 to 10, R is hydrogen, $n$ has a value of from 12 to 22, more preferably 14 to 20, $x$ has a value of at least 10, more preferably from 10 to 50, most preferably from 15 to 30, $y$ has a value from 1 to 10, more preferably 1 to 5, most preferably 1 to 4, $z$ has a value of from 10 to 30, more preferably 12 to 25, most preferably 14 to 20, $x+y+z$ has a value of from 25 to 100, more preferably 30 to 90, most preferably 35 to 80.

Examples of useful copolymers according to the invention may have the following parameters:

| a | p | q | R | n | x | y | z |
|---|---|---|---|---|---|---|---|
| 2 | 7 | 0 | H | 16 | 50 | 18 | 4 |
| 2 | 12 | 0 | H | 12 | 20 | 12 | 2 |
| 3 | 7 | 0 | H | 18 | 50 | 18 | 2 |
| 3 | 12 | 0 | H | 8 | 20 | 15 | 1 |
| 3 | 7 | 0 | $CH_3$ | 18 | 10 | 10 | 2 |

The copolymers according to the invention can be prepared by well known processes, many of which have been described in a number of publications. The copolymers may be prepared by methods similar to those described in the above mentioned prior art. One particularly preferred method of making them comprises a first step of preparing a silicone hydride functional polydimethyl siloxane by ring-opening co-polymerisation of methylhydrogen polysiloxane, cyclic dimethyl siloxane and end-blocking siloxanes in the presence of a suitable acidic catalyst, followed by a second step of reacting via addition reaction an unsaturated $C_{8-24}$ alkene in the presence of a Group VIII catalyst, e.g. a platinum based catalyst, and a third step of reacting via addition reaction an alkenyl ended polyoxyalkylene polymer. Steps 2 and 3 may by done simultaneously. It is of course important to provide the desired ratios of reagents and catalysts, and where necessary to remove or inactivate the catalyst at the end of the process.

The copolymer according to the invention is particularly useful for providing stable water-in-oil emulsions, particularly where the oil phase does include or is mainly composed of polar oil. They are however also suitable for use in other water-in-oil emulsions.

According to a second aspect of the invention, there is provided a water-in-oil emulsion, comprising a discontinuous aqueous phase, a continuous oil phase and an organopolysiloxane polyoxyalkylene copolymer of the general formula

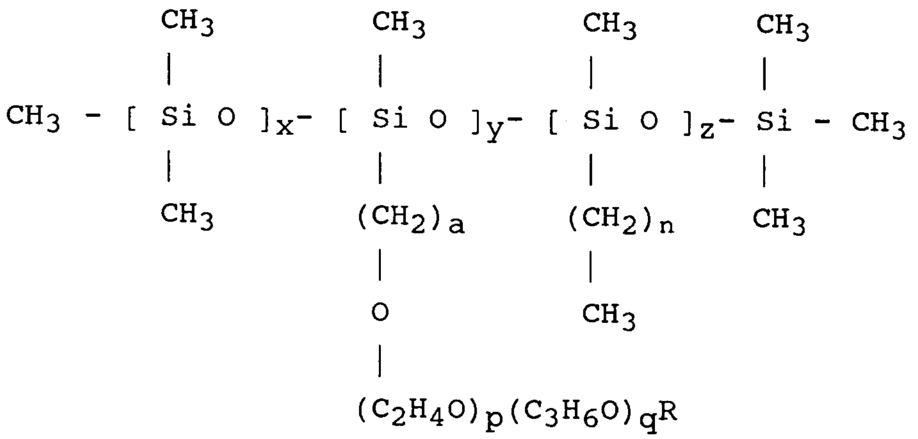

(I)

wherein R denotes a hydrogen atom or a $C_{1-4}$ alkyl group, x, y and z have a value of at least 1, provided that x+y+z has a value of from 20 to 150, 3y must be greater than x, a has a value of from 1 to 3, p has a value of at least 1 and q is 0 or an integer, provided that p and q are such that the molecular weight of $(C_2H_4O)_p(C_3H_6O)_qR$ is no more than 500 and n has a value of from 7 to 23.

The discontinuous aqueous phase comprises water, and may also comprise water-soluble components, e.g. electrolytes, preferably cosmetically acceptable electrolytes, which will enhance the stability of the emulsion, especially in the case of free-flowing emulsions. Typical examples of acceptable electrolytes include sodium chloride, sodium acetate, sodium citrate, sodium sulphate, sodium phosphate, ammonium carbonate and magnesium sulphate.

The continuous oil phase may contain any oil, preferably cosmetically acceptable oils and other oil compatible ingredients. Examples of acceptable oils include hydrocarbon oils and waxes, e.g. mineral oil, petrolatum, perhydrosqualene, paraffin wax; silicone oils, e.g. cyclic and linear polydimethylsiloxanes, polyphenylmethylsiloxanes, methylsiloxane resins and mixtures thereof; esters, e.g. lanolin, triglyceride esters, fatty esters of glycols, glycerol, sorbitol and mannitol, alkyl esters of fatty acids such as methyl esters of fatty acids; non-volatile alcohols, e.g. cholesterol, lanolin alcohol, lauryl alcohol, cetyl alcohol, oleyl alcohol and stearyl alcohol; phospholipids; fatty alcohol ethers, e.g. stearyl ether of polyoxyethylene. However, it is preferred that the continuous oil phase comprises polar oils, e.g. vegetable based oils, olive oil, castor oil, soybean oil, sunflower oil, avocado oil, jojoba oil, coconut oil, wheat germ oil, palmitic acid or triglycerides, e.g. caprilic/capric triglyceride.

The copolymer used in the emulsion according to the invention has been described above, and may be used alone, or in combination with other emulsifiers, either silicone-based ones or pure organic ones. It is however preferred that only the copolymer according to the invention is used as the emulsifier in the emulsions according to the invention. The emulsifier is preferably used in amounts up to 10% by weight based on the total weight of the emulsion. More preferably from 1 to 5% is used, most preferably from 2 to 3%.

Additional ingredients may be included in the oil or aqueous phase, e.g. barrier agents, humectants, sunscreens, vitamins, hormones, fillers, colorants, pigments, perfumes, preservatives, liposomes, salts, proteins, alcohol and glycerine.

It was surprisingly found that in emulsions according to the invention substantial quantities of oils could be incorporated, without affecting the stability of the emulsion. This is particularly advantageous for polar oils, which cannot normally be included in such high level. The emulsions according to the invention may contain up to 50% by weight of the oil phase, although for improved stability, it is preferred that the emulsions comprise up to 30% by weight of the oil phase. More preferably up to 25% is used. When measured as phase volume ratio, up to 45% of the oil phase has been successfully incorporated. Higher amounts are possible when the oil phase is more polar.

The emulsions are prepared in known ways, using standard techniques. Examples of methods include pure mechanical emulsification, for example using a colloid mill or high shear mixer, ultrasonic emulsification, e.g. using a sonolator. Where waxy or solid ingredients are included, it may be necessary to heat the ingredients in order to facilitate the emulsification.

Emulsions according to the invention may be any water-in-oil emulsions, but are preferably used in the cosmetic industry. They may take the form of lotions or creams or any other suitable form. Emulsions according to the invention may also be used for other applications, including for industrial applications such as well drilling fluids, textile emulsions, fibre treatment emulsions for the paper industry.

There now follow a number of examples which illustrate the invention, in which all parts and percentages are given by weight, unless otherwise indicated.

Preparation of copolymers

**Example 1**

33 parts of a trimethylsilyl end-blocked methylhydrosiloxane having an average chainlength of about 50 siloxane units, 65.5 parts of a mixture of octamethylcyclotetrasiloxane and decamethylcyclopentasiloxane and 1.44 parts of hexamethyldisiloxane were heated to 70°C in the presence of a sulphonic acid catalyst (0.1%) for 5 hours in a flask. After cooling and neutralising with $NaHCO_3$, the mixture was filtered and residual low volatility materials were removed by stripping at reduced pressure. The resulting polymer had the average structure $(CH_3)_3SiO[(CH_3)_2SiO]_{50}[CH_3HSiO]_{30}Si(CH_3)_3$. To 39 parts of this polymer sufficient of a platinum catalyst was added to give 2.5 $10^{-5}$ mole per mole of SiH in the polymer. 22 parts of 1-hexadecene were added and the mixture held under vacuum at 100°C for 2 hours. After allowing the mixture to cool to 60°C, 9 parts of isostearyl alcohol and 8 parts of allylethylene glycol were added, and the mixture reheated to 100°C after addition of addition platinum catalyst. After 30 minutes, another 23 parts of 1-hexadecene were added with some additional platinum, to give after cooling and filtration a polymer of the formula

```
           CH3         CH3          CH3      CH3
           |           |            |        |
   CH3 - [ Si O ]50- [ Si O ]27- [ Si O ]3- Si - CH3    (I)
           |           |            |        |
           CH3       (CH2)3      (CH2)15    CH3
                        |            |
                     O(C2H4O)7H     CH3
```

**Example 2-10**

Similar copolymers were prepared using the same process and adjusted amounts of ingredients according to Example 1, resulting in copolymers of the formula (I) with the following parameters

| Example | a | p | q | R | n | x | y | z |
|---|---|---|---|---|---|---|---|---|
| 2 | 3 | 7 | 0 | H | 15 | 50 | 18 | 2 |
| 3 | 3 | 7 | 0 | H | 15 | 20 | 18 | 2 |
| 4 | 3 | 7 | 0 | H | 15 | 10 | 18 | 2 |
| 5 | 3 | 7 | 0 | H | 15 | 20 | 16 | 4 |
| 6 | 3 | 7 | 0 | H | 15 | 20 | 19 | 1 |
| 7 | 3 | 7 | 0 | H | 15 | 20 | 27 | 3 |
| 8 | 3 | 7 | 0 | H | 15 | 10 | 27 | 3 |
| 9 | 3 | 7 | 0 | H | 15 | 20 | 24 | 6 |
| 10 | 3 | 7 | 0 | H | 15 | 20 | 28.5 | 1.5 |

**Comparative Example 1**

In order to compare the efficiency of the copolymers according to the invention, a silicone-based emulsifier, known for its efficiency in emulsifying mineral oils was used. This emulsifier was prepared according to the teaching of Example 1 of US patent 4,853,474.

Preparation of emulsions

The process used for making the following emulsions was substantially the same for all emulsions, except that where solid materials were used, an additional heating step was included to ensure the ingredients were used in a liquid form during the process. All water phase ingredients were mixed until homogeneous together with the copolymer emulsifier, all oil phase ingredients were mixed separately. Then the water phase was slowly added to the oil phase under strong agitation, followed by passing the mixture through a homogeniser, in this case a Silverson® homogeniser was used.

**Examples 11-20**

Each of copolymers of Examples 1 to 10 were used in an emulsion comprising 25% sunflower oil, 2% of the copolymer and 73% water. The emulsions were measured for viscosity, and for stability by storing them at room temperature, i.e. 20°C (RT) and at 40°C (40) for up to 2 months (M). Table I below shows the resulting viscosity and the stability results.

TABLE I

| Example Emulsion | Copolymer from Ex. | viscosity mPa.s (25°C) | Stability |
|---|---|---|---|
| 11 | 1 | 153,000 | 1M 40 / 1M RT |
| 12 | 2 | 184,000 | 1M 40 / 1M RT |
| 13 | 3 | 158,000 | 2M 40 / 2M RT |
| 14 | 4 | 113,000 | 1M 40 / 2M RT |
| 15 | 5 | 123,000 | 1M 40 / 1M RT |
| 16 | 6 | 132,000 | 2M 40 / 2M RT |
| 17 | 7 | 149,000 | 2M 40 / 2M RT |
| 18 | 8 | 190,000 | 1M 40 / 2M RT |
| 19 | 9 | 67,400 | 1M 40 / 2M RT |
| 20 | 10 | 133,000 | 1M 40 / 2M RT |

**Example 21**

In this example only percentages are given in volume, not in weight. Emulsions were prepared according to the method outlined above. The oil phase consisted of either mineral oil (M), caprylic/capric triglycerides (G) or sunflower oil (S), which was incorporated in varying amounts. As emulsifiers only Examples 1 and 2 were used at a constant 2% incorporation. Comparative emulsions were prepared with the emulsifier of comparative example 1. The result of the examples what that stable emulsions could be prepared with up to 25% of M oil for Example 1 copolymer, 22% for copolymer 2, and 25% for Comparative Example 1 copolymer. For oil G, up to 32% could be used with Example 1 copolymer, 27% with Example 2 copolymer and 27% with the Comparative Example 1 copolymer. For oil S, Example 1 copolymer allowed the use of 35% oil, Example 2 copolymer the use of 30% oil, while Comparative Example 1 copolymer could not give any stable emulsions at all.

**Example 22**

Emulsions were prepared using the ingredients of Example 21, including the comparative example copolymer, in order to show the amount of copolymer according to the invention which was required to give a stable emulsion. For 22% oil M, Example 1 and Example 2 copolymers were needed at 0.75%, whereas Comparative Example 1 copolymer was needed at 1%. For 24% of (G) oil, Example 1 copolymer was needed at 1.5%, Example 2 copolymer at 1% and Comparative Example 1 copolymer at 2%. For 27% F oil, only 2% was needed of each of Example 1 or Example 2 copolymers, whereas no stable emulsions could be made with Comparative Example 1 copolymer.

**Examples 23-35**

Stable emulsions were prepared according to the process given above. Ingredients are mentioned with the percentage used, in each case the balance is water (up to 100%). It will be obvious to the person skilled in the art which ingredients belong to the oil phase, and which belong to the aqueous phase. The person skilled in the art will also easily recognise which ingredients require additional heating in the preparation of the emulsions. The emulsions were tested for stability and viscosity, and were satisfactory in all respects.

Ex.23

| | |
|---|---|
| Caprilic/capric triglyceride | 10 |
| castor oil | 10 |
| mineral oil | 5 |
| example 1 or 2 copolymer | 2 |
| glycerine | 3 |
| NaCl | 1 |

Ex.24

| | |
|---|---|
| caprylic/capric triglycerides | 15 |
| castor oil | 10 |
| example 1 or 2 copolymer | 2 |
| glycerine | 3 |
| NaCl | 1 |

Ex.25

| | |
|---|---|
| isopropyl palmitate | 20 |
| example 1 or 2 copolymer | 2 |
| glycerine | 3 |
| NaCl | 1 |

Ex.26

| | |
|---|---|
| olive oil | 25 |
| example 1 or 2 copolymer | 2 |
| glycerine | 3 |
| NaCl | 1 |

Ex.27

| | |
|---|---|
| soybean oil | 25 |
| example 1 or 2 copolymer | 2 |
| glycerine | 3 |
| NaCl | 1 |

Ex.28

| | |
|---|---|
| example 1 or 2 copolymer | 2 |
| octyl methoxycinnamate | 6 |
| butyl methoxydibenzoylmethane | 1 |
| benzophenone-3 | 3 |
| $C_{12-15}$ alkyl benzoate | 6 |
| coco caprylate/caprate | 3 |
| parabens | 0.3 |
| Dow Corning® 1401 | 4 |
| NaCl | 1 |
| Glycerine | 5 |
| EDTA Na | 0.1 |

Ex.29

| | |
|---|---|
| example 1 or 2 copolymer | 2 |
| sunflower oil | 5 |
| Dow Corning® 2503 | 3 |
| Dow Corning® 2502 | 5 |
| Dow Corning® 5513 | 5 |
| caprylic/capric triglycerides | 8 |
| NaCl | 1 |
| glycering | 3 |

Ex.30

| | |
|---|---|
| example 1 or 2 copolymer | 2 |
| shea butter | 2 |
| avocado oil | 4 |
| jojoba oil | 4 |
| Dow Corning® 345 | 5 |
| $C_{12-15}$ alkyl benzoate | 5 |
| NaCl | 1 |
| glycerine | 3 |

Ex.31

| | |
|---|---|
| example 1 or 2 copolymer | 2 |
| caprylic/capric triglycerides | 10 |
| sunflower oil | 5 |
| ispropyl palmitate | 4 |
| vitamin E acetate | 1 |
| sorbitol | 7 |
| silk amino acids | 3 |
| glycerine | 5 |
| urea | 2 |

Ex.32

| | |
|---|---|
| example 1 or 2 copolymer | 2 |
| caprylic/capric triglycerides | 5 |
| sunflower oil | 5 |
| ispropyl palmitate | 10 |
| NaCl | 1 |
| Ethanol | 20 |

Ex.33

| | |
|---|---|
| example 1 or 2 copolymer | 2 |
| coconut monoisopropanolamide | 4 |
| Crothix® LVR | 2 |
| sodium Lauryl sulphate (30%) | 30 |

Ex.34

| | |
|---|---|
| example 1 or 2 copolymer | 2 |
| $C_{12-15}$ alkyl benzoate | 5 |
| isoparaffin | 5 |
| Dow Corning® 344 | 10 |
| aluminium chlorohydrate (50%) | 38 |

Ex.35

| | |
|---|---|
| example 1 copolymer | 2 |
| FeO2 black | 0.064 |
| FeO2 yellow | 0.54 |
| FeO2 red | 0.22 |
| TiO2 | 8 |
| aluminium hydroxy stearate | 5 |
| caprylic/capric triglycerides | 5 |
| Dow Corning® 345 | 15 |
| Dow Corning® 2502 | 5 |
| NaCl | 1 |
| Glycerine | 3 |

## Claims

1. An organopolysiloxane polyoxyalkylene copolymer of the general formula

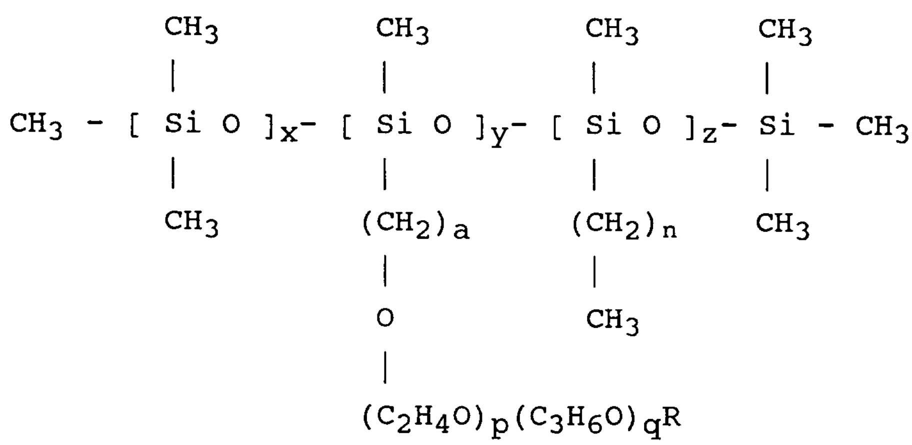

wherein R denotes a hydrogen atom or a $C_{1-4}$ alkyl group, x, y and z have a value of at least 1, characterised in that x+y+z has a value of from 20 to 150, 3y is greater than x, a has a value of from 1 to 3, p has a value of at least 1 and q is 0 or an integer, the molecular weight of $(C_2H_4O)_p(C_3H_6O)_qR$ is no more than 500 and n has a value of from 7 to 23.

2. A copolymer according to claim 1, further characterised in that a has a value of 3 and q has a value of 0.

3. A copolymer according to Claim 1 or 2 further characterised in that p has a value of from 4 to 15.

4. A copolymer according to any one of the preceding claims, further characterised in that n has a value of from 12 to 22.

5. A copolymer according to any one of the preceding claims, further characterised in that x has a value of at least 10, y has a value from 1 to 10 and z has a value of from 10 to 30.

6. A copolymer according to any one of the preceding claims, further characterised in that $x+y+z$ has a value of from 25 to 100.

7. A water-in-oil emulsion, comprising a discontinuous aqueous phase, a continuous oil phase and an organopolysiloxane polyoxyalkylene copolymer characterised by the general formula

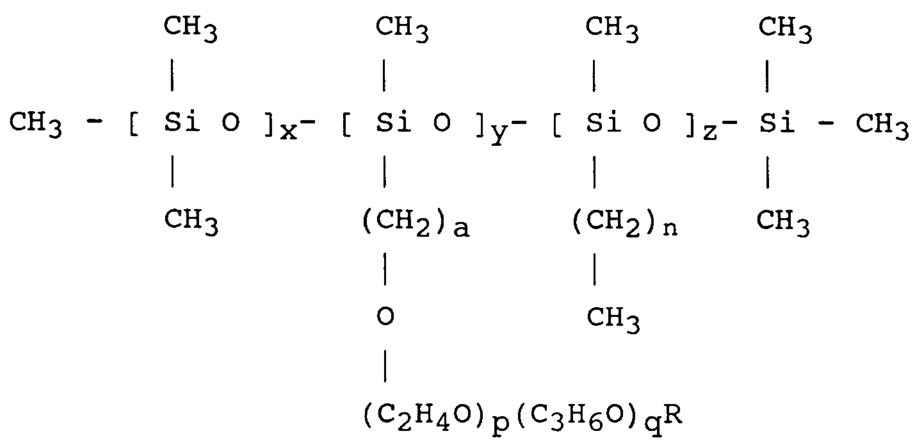

wherein R denotes a hydrogen atom or a $C_{1-4}$ alkyl group, $x$, $y$ and $z$ have a value of at least 1, $x+y+z$ has a value of from 20 to 150, $3y$ is greater than $x$, $a$ has a value of from 1 to 3, $p$ has a value of at least 1 and $q$ is 0 or an integer, the molecular weight of $(C_2H_4O)_p(C_3H_6O)_qR$ is no more than 500 and $n$ has a value of from 7 to 23.

8. A water-in-oil emulsion according to Claim 7 further characterised in that the discontinuous aqueous phase comprises water, and water-soluble components.

9. A water-in-oil emulsion according to Claim 7 or 8 further characterised in that the continuous oil phase comprises polar oils.

10. A water-in-oil emulsion according to any one of Claims 7 to 9 further characterised in that the continuous oil phase comprises an oil selected from olive oil, castor oil, soybean oil, sunflower oil, avocado oil, jojoba oil, coconut oil, wheat germ oil, palmitic acid or triglycerides.

11. A water-in-oil emulsion according to any one of Claims 7 to 10 further characterised in that the copolymer is used in amounts of from 1 to 5% by weight.

12. A water-in-oil emulsion according to any one of Claims 7 to 11 further characterised in that it contains up to 25% by weight of the continuous oil phase.